# EUROPEAN PATENT SPECIFICATION

(11) **EP 1 527 201 B1**
(45) Date of publication and mention of the grant of the patent: **21.02.2007**
(21) Application number: 03766454.7
(22) Date of filing: 31.07.2003
(51) Int. Cl.: C12Q 1/68

(54) **ANALYSIS OF BIOLOGICAL SAMPLES**
ANALYSE BIOLOGISCHER PROBEN
ANALYSE D'ECHANTILLONS BIOLOGIQUES

(30) Priority: 03.08.2002 GB 0218087
(43) Date of publication of application: 04.05.2005
(73) Proprietor: EPISTEM LIMITED, Manchester M13 9XX (GB)
(72) Inventor: BRADY, Gerard, Charlesworth, Glossop SK13 5DD (GB)
(74) Representative: Armstrong, Iain Cheshire
(86) International application number: PCT/GB2003/003322
(87) International publication number: WO 2004/013352

(56) References cited:
- US-B1- 6 265 163
- WELCHER A A ET AL: "SELECTIVE ENRICHMENT OF SPECIFIC DNA, CDNA AN RNA SEQUENCES USING BIOTINYLATED PROBES, AVIDIN AND COPPER-CHELATE AGAROSE" NUCLEIC ACIDS RESEARCH, OXFORD UNIVERSITY PRESS, SURREY, GB, vol. 14, no. 24, 1986, pages 10027-10044, XP002051197 ISSN: 0305-1048
- ELENITOBA-JOHNSON KOJO S J ET AL: "Solution-based scanning for single-base alterations using a double-stranded DNA binding dye and fluorescence-melting profiles" AMERICAN JOURNAL OF PATHOLOGY, vol. 159, no. 3, September 2001 (2001-09), pages 845-853, XP002259706 ISSN: 0002-9440
- LYON E: "Mutation detection using fluorescent hybridization probes and melting curve analysis." EXPERT REVIEW OF MOLECULAR DIAGNOSTICS. ENGLAND MAY 2001, vol. 1, no. 1, May 2001 (2001-05), pages 92-101, XP002259707 ISSN: 1473-7159

### SEQUENCE LISTING

<110> Epistem Limited
   Brady, Gerard
<120> ANALYSIS OF BIOLOGICAL SAMPLES
<130> P089181PWO
<160> 3
<170> PatentIn version 3.1
<210> 1
   <211> 40
   <212> DNA
   <213> Unknown
<220>
   <223> Not1dT - oligonucleotide primer
<400> 1
   catctcgagc ggccgctttt tttttttttt tttttttttt 40
<210> 2
   <211> 22
   <212> DNA
   <213> Unknown
<220>
   <223> Display oligo A - oligonucleotide primer
<400> 2
   cagccagtct tgaggcaaca cc 22
<210> 3
   <211> 24
   <212> DNA
   <213> Unknown
<220>
   <223> Display oligo B - oligonucleotide primer.
<400> 3
   ccagcaagag cacaagagga agag 24

### SEQUENCE LISTING

<110> Epistem Limited
   Brady, Gerard
<120> ANALYSIS OF BIOLOGICAL SAMPLES
<130> P089181PWO
<160> 3
<170> PatentIn version 3.1
<210> 1
   <211> 40
   <212> DNA
   <213> Unknown
<220>
   <223> Not1dT - oligonucleotide primer
<400> 1
   catctcgagc ggccgctttt tttttttttt tttttttttt 40
<210> 2
   <211> 22
   <212> DNA
   <213> Unknown
<220>
   <223> Display oligo A - oligonucleotide primer
<400> 2
   cagccagtct tgaggcaaca cc 22
<210> 3
   <211> 24
   <212> DNA
   <213> Unknown
<220>
   <223> Display oligo B - oligonucleotide primer.
<400> 3
   ccagcaagag cacaagagga agag 24

## Description

The present invention relates to a method of determining the degree of similarity between nucleic acid content of a biological sample of interest and that of individual reference samples. The method is particularly suitable for comparing gene expression between a sample of interest and reference samples.

Recent years have seen a growth in the realisation of the importance of gene expression in the control of biological activities. It is known that expression of specific subsets of genes regulate tissue formation and organogenesis during development and also the properties of adult tissues. Patterns of gene expression influence not only the structure and composition of specific tissues, but also the tissues' responses to various stimuli. These structures, composition and responses, and the patterns of gene expression encoding them, are distinctive markers for individual tissues.

At a more complex level the pattern of genes expressed by whole organisms may be characteristic of specific individuals and provide an insight into their biological status. For instance, there is growing evidence that the pattern of genes expressed by an individual may influence factors such as the individual's predisposition to particular diseases or their responsiveness to certain therapeutic agents.

The current challenge to biologists is to learn how the products of the around 40,000 identified human genes interact to produce the complexity exhibited by higher eukaryotes. To a large extent the biological character of a cell can be inferred from the profile of genes it expresses. Although an examination of mRNA or protein expression patterns alone does not directly address function, the knowledge of when and where a gene is expressed can provide valuable insights as to the potential role of a gene and has historically been instrumental in the discovery of developmentally regulated genes. Recognition of the value of the examination of expression patterns led to the development of a plethora of advanced mRNA profiling technologies such as cDNA microarrays (Duggan et al., 1999), SAGE (Velculescu et al., 1995), and cDNA display (Liang and Pardee, 1992) aimed at the simultaneous measurement of tens to several thousand genes in the target samples. Application of these profiling technologies to clinical diseases, such as cancer has confirmed the utility of profiling and provided useful diagnostic and prognostic assays (Shipp et al., 2002; Staunton et al., 2001; van 't Veer et al., 2002).

Despite the success of these approaches at the molecular level by identifying patterns of expression exhibited generally by relatively homogeneous cellular samples the cellular complexity of higher eukaryotes still presents a major obstacle to expression profiling.

Over the last 30 years a variety of molecular techniques have been developed for the analysis of gene-expression. In general methods focussed either on the identification and characterisation of genes (either individual genes or networks of related genes) or the characterisation of the input tissue or cell based on a characteristic profile of expressed genes. Although conventional nucleic acid hybridization techniques (such as northern and dot blots) have been used for many years to analyse a small number of genes and samples there have been a variety of advanced mRNA profiling technologies such as cDNA microarrays (Duggan et al., 1999), SAGE (Velculescu et al., 1995), and cDNA display (Liang and Pardee, 1992) which have been recently developed to allow the simultaneous measurement of tens to several thousand genes in the target samples. In order to take both full advantage of and to extend recent improvements in gene-expression analysis it is important that the final processed sample in the form of RNA or cDNA is compatible with a wide variety of expression profiling methods.

It is an object of the present invention to obviate or mitigate the disadvantages associated with the prior art.

According to the present invention there is provided a method of determining the degree of similarity between nucleic acid content of a biological sample of interest and that of individual reference samples, comprising
(a) providing a nucleic acid probe library representative of nucleic acid content of the biological sample of interest,
(b) providing a plurality of reference samples each being a nucleic acid library representative of nucleic acid content of reference biological samples from which the reference samples have been derived,
(c) forming a first set of immobilised, hybridised products by treating the individual reference samples with the probe library under hybridising conditions, one or other of the reference samples or the probe library being in immobilised form, and removing non-immobilised material,
(d) forming a second immobilised product by treating a sample of the free probe library with an immobilised sample of the probe library under hybridising conditions, and removing non-immobilised material,
(e) effecting progressive dissociation of the hybridised products obtained in steps (c) and (d),
(f) monitoring said progressive dissociation, and
(g) comparing the results of step (f) for the hybridised products obtained in step (c) with those obtained for the hybridised products obtained in step (d) to determine said degree of similarity.

The present invention makes it possible to obtain an indication of the degree of similarity between the nucleic acid content of a sample of interest and that in a number of reference samples. The greater the degree of similarity between the nucleic acid content of the sample of interest and the nucleic acid content of a particular reference sample then the greater the similarity between these two samples. The ability to compare nucleic acid content of a sample of interest with that in a large number of reference samples is, of course, particularly advantageous where the reference samples are of well characterised biological status since conclusions may then be drawn as to the biological status of the sample of interest.

The method of the invention is applicable to all forms of nucleic acid, such as genomic DNA, cDNA, mRNA and tRNA. However, the method is particularly suited to comparison of nucleic acids representative of gene expression in biological samples. Nucleic acids representative of gene expression may, for example, comprise mRNA, cDNA, or derivatives thereof. Although the invention is described below, for illustrative purposes, in terms of comparison of nucleic acids representative of gene expression it should be appreciated that the invention is applicable to comparison of other nucleic acid populations.

The method of the invention utilises a nucleic acid probe library representative of the nucleic acid content of the sample of interest. The individual reference samples are each libraries comprised of nucleic acid representative of the nucleic acid content of the biological samples from which the reference samples are derived.

A library for use according to the method of the invention is a collection of individual nucleic acid sequences representative of the population of nucleic acid within the biological sample from which the library is derived. The nucleic acid may comprise any form of nucleic acid, such as DNA (including subtypes of DNA such as cDNA) and RNA (including subtypes of RNA such as mRNA and tRNA). The number of sequences in the collection is sufficient to provide significant information about the biological activity or status of the biological sample from which the library is derived. Although a library may represent all nucleic acids present in a biological sample this need not be the case. Indeed it may be preferred that a library represent only nucleic acid characteristic of a biological sample. Thus (in the case of nucleic acids representative of gene expression), although a biological sample may express many thousands of genes a library may, for instance, represent expression of ten or more genes the expression of which are characteristic of the activity or status of the biological sample. Preferably a library representative of gene expression may represent twenty or more genes, and most preferably fifty or more genes, the expression of which are characteristic of the activity or status of the biological sample. This does not, however, preclude the possibility that a library representative of gene expression may represent all genes expressed by a biological sample.

In order to effect the method of the invention a first set of immobilised hybridised products is produced by treating the reference samples with the probe library under hybridising conditions. The immobilisation of the hybridised products is achieved by immobilising one or other of the probe library or reference samples to a substrate prior to hybridisation. Non immobilised material, that is to say material that is not attached to the substrate or has not hybridised to material attached to the substrate, is then removed. This removal may be effected by washing. A second set of immobilised, hybridised products is produced by treating a free sample of the probe library with an immobilised sample of the probe library (i.e. a sample of the probe library that is attached to a substrate). Non-hybridised material is, once more, removed.

The hybridised materials from the first and second immobilised products are then subjected to progressive dissociation. Dissociation may be effected whilst the products remain immobilised or alternatively after the hybridised products have been removed from the substrate. The progressive dissociation of the hybridised products is monitored. This produces separate dissociation patterns representing dissociation of the hybridised materials in the different reference samples of the first immobilised product, and a further dissociation pattern representing dissociation of the hybridised material of the second immobilised product.

The dissociation pattern of the hybridised material of the second immobilised product represents the dissociation of probe library material that has undergone hybridisation with other probe library material, that is to say probe library material that has hybridised with "itself". By comparing this dissociation pattern to the dissociation patterns obtained from dissociation of the probe library and individual reference samples, it is possible to identify which of the individual reference samples is/are most similar to the probe library (i.e. the reference sample(s) which have a dissociation pattern most similar to the dissociation pattern obtained from the de-hybridisation of the material from the second immobilised product).

More particularly different hybrids, representing different nucleic acids in the sample of interest, will dissociate from one another at different degrees of dissociating conditions. The dissociation of the samples can be monitored as a means of providing further information about the nature of the nucleic acid molecules common to the sample of interest and the reference samples. This method may be used to distinguish dissociation "signatures" specific to particular sequences.

Progressive dissociation of the hybridised samples may be brought about by exposing the samples to increasing temperature. Alternatively the dissociation may be promoted by increasing concentrations of chemical denaturants.

Monitoring the dissociation of the hybridised nucleic acid samples may be effected by a number of different techniques.

According to one suitable embodiment the samples may be labelled with a marker capable of differentiating between double-stranded and single-stranded nucleic acids. Such a marker may be a fluorescent marker. For example, in the case where the nucleic acid samples comprise DNA, the marker may be an intercalating dye such as ethidium bromide. An alternative dye capable of differentiating between double and single-stranded DNA is the commercially available fluorescent marker SybrGreen (Molecular Probes, Oregon, USA), the fluorescence of which increases in the presence of double stranded DNA. SybrGreen is the proprietary name for an unsymmetrical cyanine dye (CAS No.: 163795-75-3). This embodiment is suitable for monitoring dissociation both of immobile hybridised populations and of hybridised populations in free solution.

In an alternative embodiment progressive dissociation may be monitored by detecting the generation of single stranded nucleic acids on de-hybridisation of the double stranded hybridised material. If un-hybridised material is removed on completion of hybridisation, then single stranded nucleic acid molecules will only be present as a result of their generation by dissociation of hybridised material. Detection of single stranded nucleic acids may be achieved by using appropriate specific reagents. Single stranded DNA, for example, can be detected using single stranded DNA binding protein (SSB) (Sigal, et al. 1972; Williams, et al. 1983).

In a different embodiment the non-immobilised nucleic acid population (either the probe library or reference samples) may be labelled with a marker, and the immobilised nucleic acid population unlabelled. Thus each hybridisation event involving nucleic acid molecules of the immobilised population will contain both labelled and unlabelled nucleic acid molecules. When non-immobilised material is removed (including all non-hybridised labelled nucleic acids) the only labelled nucleic acid molecules that remain will be those hybridised with nucleic acids of the immobilised population. The presence of the marker is thus a specific indicator of hybridised material. As progressive dissociation occurs labelled material detaches from the immobilised material and may then be removed. Dissociation of the hybridised molecules therefore causes the progressive loss of the marker from the immobilised material. Dissociation can be monitored by assessing the residual label retained by the immobilised material on removal of non-immobilised material, or by assessing the labelled material released from the immobilised material. Markers suitable for use with this embodiment of the invention include fluorescent and radioactive labels, and chromogenic enzymes such as horseradish peroxidase.

Alternatively both the probe library and reference samples may be labelled with markers capable of generating a signal when the markers are in proximity to one another that can be distinguished from that signal generated when the markers are distant from one another. Suitable markers for use in such an embodiment include fluorescent markers capable of forming Fluorescent Resonance Energy Transfer (FRET) partners (i.e. a FRET donor and a FRET acceptor). Examples of suitable FRET partners are well known to those skilled in the art.

A preferred embodiment of the invention employs two different methods of monitoring dissociation. Such an embodiment may, for example, utilise both a marker capable of differentiating between double-stranded and single-stranded nucleic acids and a labelled non-immobilised nucleic acid population.

Dissociation data can be analysed and quantified using commercially available tools such as the ABI 7000 Sequence Detection System and the 'Dissociation Curve Analysis' program (Applied Biosystems).

Thus in a preferred embodiment hybridised DNA of a sample of interest and a reference sample labelled with the fluorescent marker SybrGreen is exposed to incrementally increasing temperature. As the temperature increases the decrease in hybridisation of the DNA samples causes a corresponding decrease in fluorescence from the SybrGreen. The dissociation curve thus produced can be analysed using commercially available software in order to further investigate the DNA shared by the sample of interest and the reference sample. For example it is known that hybrids representing certain genes dissociate at particular temperatures (so called high temperature and low temperature genes) and analysis of the dissociation curve will provide information as to what proportion of high and low temperature genes are shared by the samples. This information can then be compared with data generated by the same method using different reference samples in order to produce further characterising information relating the probe library to the individual reference samples. For instance if a condition is known to be associated with the expression of a gene (or genes) that dissociate at characteristic temperature(s) then complexity analysis may be able to indicate whether or not hybridisation of that (or those) particular gene (or genes) represents a component of the total hybridisation between a sample of interest and a reference sample.

The ability to compare overall gene expression in a sample of interest with that in a number of reference samples is particularly advantageous when the reference samples are of defined and well-characterised biological status since conclusions may then be drawn as to the biological status of the sample of interest.

The method of the invention need only require a single round of hybridisation to allow comparison between the pattern of expression of a plurality of genes in a sample of interest with the pattern of expression of the same genes in a number of reference samples. The pattern of expression may potentially extend to the expression of thousands of different genes. Since known techniques only analyse the expression of either small numbers of genes or small numbers of samples such information could only be provided by the prior art on completing multiple rounds of hybridisation. Thus the method of the invention provides advantages both in terms of a reduction in the time necessary to perform such a comparison, and also in the reduced amount of reagents required.

In contrast to existing methodologies (in which specific probes are used to investigate the expression of specific genes) the method of the invention is able to compare patterns of gene expression without requiring any specific information as to the genes involved. Thus it is not necessary to identify those genes that may be of interest before comparing patterns of gene expression between unknown and reference samples. This provides a considerable advantage over the prior art in that an investigator does not need to know what gene (or genes) are involved in, for example, a particular response to a therapeutic agent before he can establish whether a test subject is likely to respond in a similar way to a previously characterised subject with a known response.

The reference samples may be derived from biological reference samples representing a number of different biological conditions or states. Alternatively the reference samples may be derived from biological reference samples representing a number of different examples of the same biological condition or state. Individual reference samples may be derived from biological samples taken from one or more individual. In the instance that a reference sample is derived from a single individual the reference sample may be derived from a biological sample representing a single tissue, or from biological samples representing a number of different tissues. In the case of reference samples derived from biological samples taken from more than one individual the biological samples may all represent one type of tissue, or may represent a number of different tissue types. By including reference samples which share a common biological phenotype yet have arrived at that state via different routes the method of the invention is able to discriminate between treatment and biological status.

Examples of materials that may be used as reference samples include samples which are derived from patients with known clinical conditions and/or with known clinical outcomes.

In one such example reference samples may be taken from a number of patients with different forms of a particular disease. In this instance the sample of interest may be taken from an individual suspected of having, or being predisposed to, the disease in question. By comparing the pattern of gene expression in the sample of interest with the patterns of gene expression in the reference samples it is possible to establish which of the reference samples the sample of interest most closely resembles. This may then in turn provide an indication as to the particular form of the disease in question that the individual tested has or is predisposed to.

Alternatively the reference samples may be derived from patients with the same disease, but having different (known) reactions to different therapeutic agents. In this case comparison of the sample of interest and the reference samples will establish which of the patients with known treatment history the individual providing the sample of interest most resembles. This knowledge can then be used in order to select the treatment regime believed most likely to produce a beneficial outcome for the individual in question.

In another alternative reference samples may be derived from the same patient at different times, for instance before, during and after therapy. Comparison of such samples with a sample of interest taken from a patient with the same disease may be useful in assessing the progress of the patient of interest during treatment.

In a further alternative suitable reference samples may be collected from experimental subjects, such as animals or cultured cells, that have undergone procedures the effects of which are well studied.

For example reference samples may be collected from cells of a cell line that have been exposed to different drugs that have known effects (either on the cell line or on organisms of interest). These samples may then be probed using a probe library derived from cells of the same cell line that have been exposed to a putative drug which has an unknown effect. By comparing gene expression patterns established in response to the known and unknown drugs it is then possible to establish which of the known drugs the unknown drug most resembles. This will provide an indication that the effects of the unknown drug are likely to be similar to those of the known drug that it most closely resembles.

In a further example the sample of interest may be taken from tissues of experimental animals that have undergone treatments bringing about conditions that resemble those of a disease of interest. The pattern of gene expression in these samples may then be compared with the pattern of gene expression in reference samples taken from normal biological samples or biological samples from individuals suffering from the disease in question in order to investigate how changed gene expression influences the particular disease. Suitable experimental animals may, for instance, include transgenic animals, such as animals in which certain genes have been up-regulated, down-regulated or deleted.

In another application of the method of the invention the sample of interest may be taken from a tissue that includes, or may be thought to include, a cell type of particular interest. Such cells may, for example, be stem or progenitor cells. In this case tissues representing suitable biological samples from which reference samples may be derived will include tissues known to contain the cell type of interest, or tissues known to contain specific forms of the cell type of interest. Comparison of the pattern of gene expression in the sample of interest with the pattern of gene expression in the reference samples may indicate that the sample of interest either does or does not contain the cell type of interest. If the sample of interest contains cells of the cell type of interest then the method of the invention may also provide information as to the number, form or status of these cells present in the sample.

In the field of stem cell biology defining the specific gene expression changes in stem cells, their immediate daughter cells, cells committed to differentiation and fully differentiated cells under conditions that alter self-renewal and differentiation represents a powerful means of identifying potential drug targets. For example, the discovery of a growth factor or growth factor receptor specifically expressed in stem cells undergoing increased self-renewal would lead to the development of pharmacological approaches designed to inhibit stem cell expansion during cancer development or increase stem cell expansion following injury. Furthermore, identification of genes whose expression is specifically linked to eventual stem cell self-renewal and differentiation will greatly facilitate the monitoring of stem cell behaviour that is an essential component of pre-clinical drug evaluation.

Since the method of the invention compares the patterns of expression of a number of genes within test and reference samples it is particularly well suited to the study and comparison of biological activities associated with stem cells that involve the interplay of a number of different genes, for example in a biochemical pathway. Using known methods to investigate such interactions it would be necessary to identify each gene involved in a pathway and conduct separate hybridisation reactions to determine the expression of each gene. The method of the invention, in contrast will in a single round of hybridisation report on the comparative expression of all genes involved, even including genes that may not be known to be associated with the pathway.

Conveniently the method of the invention may be effected using an array or microarray on a solid substrate. Either the probe library or the reference samples may be provided as an array or microarray on a suitable substrate. Preferably it is the reference samples that are provided as the array on the substrate. In a particularly preferred embodiment an array or microarray may comprise a library of reference samples containing DNA samples derived from groups representative of different biological conditions, each group containing samples derived from a number of different individuals sharing the same condition, wherein the DNA samples are arranged in order on the array or microarray such that members of the same group are located in proximity to one another. The DNA samples may, for instance, be arranged in order in a grid pattern such that each row of the grid represents a group of individuals sharing the same biological condition.

A suitable microarray may, for instance, be produced on a substrate such as glass, a silica-based chip, a nylon membrane or a microtiter plate. Many examples of techniques suitable for the manufacture of arrays or microarrays will be readily apparent to those skilled in the art. These include the techniques disclosed in Maniatis et al. 1982, Chee et al. 1996, Iyer et al. 1999, Lipshutz et al. 1995, Lockhart et al. 1995, Schena 1996, Schena et al. 1995, Soares et al. 1997 and Southern 1996.

DNA may be coupled to the support material forming the array by means of electrostatic interaction with a coating film of a polycationic polymer such as poly-L-lysine (as described in WO 95/35505) or may be covalently bound to the support by well established techniques.

Biological samples suitable for use according to the invention include any sample containing material representative of gene expression in the sample, such as mRNA. Biological samples preferably comprise biological cells, indeed a suitable biological sample may even comprise a single cell. Suitable samples may be taken by means of biopsies, swabs, hair or skin samples, or as samples of bodily fluids such as blood, cerebrospinal fluid (CSF), saliva, milk, faeces and urine. In particular samples for use in analysis of stem cells may suitably be taken from foetal or embryonic tissue, from bone marrow or from germ cells or from other tissues in the adult or developing organism.

The probe library and the reference samples may preferably comprise, or be derived from, global amplified cDNA, i.e. a cDNA population in which all DNA sequences are present in the same relative abundance as the mRNA from which they have been derived. Most preferably the global amplified cDNA is prepared from mRNA using limiting concentrations of nucleotides and a relatively short incubation time in order to limit cDNA synthesis. This ensures that, no matter what the length of the original mRNA transcript, all cDNA molecules produced are of approximately the same relatively small size. Since all the cDNA molecules are of approximately equal size subsequent amplification of the cDNA results in equal reproduction of all the cDNA molecules present. This ensures that the amplified cDNA produced reflects the original relative abundance of the mRNA present in the biological sample. Suitable protocols for the production of global amplified cDNA of this nature are provided in Brady *et al.* 1990, Cumano et al. 1992 and Brady *et al.* 1993. In addition to the advantage of allowing the production of amplified populations of cDNA that maintain the relative abundance of the original mRNA the use of global amplified cDNA also provides other advantages. For example global amplified cDNA can be derived either directly from one or more freshly isolated living cells without the need for RNA isolation, or from mRNA purified from a biological sample. Additionally, the production of global cDNA is well suited to automation.

Materials that may be derived from total cDNA include sub-populations of the total cDNA, truncated or otherwise manipulated versions of the cDNA and other materials representative of patterns of gene expression produced using the total cDNA as a template.

The invention may be effected using probe libraries and/or reference samples comprising cDNA produced as described above without further modification. However, various modifications may be made what will improve the sensitivity of the method. For example, cDNA, or a derivative thereof, generated from the sample of interest or the biological reference samples may be treated with a suitable 3' or 5' exonuclease.

Thus treatment of the double stranded cDNA molecules generated from the sample of interest or reference samples with exonuclease results in degradation from either the 3' or 5' end of each strand of DNA (depending on the specificity of the enzyme selected). Thus regions of each strand that are complementary to one another are removed by digestion. Digestion with double-stranded DNA (dsDNA) exonucleases will initiate digestion at each end of a double-stranded DNA molecule. Since the dsDNA exonuclease preferentially removes one strand of the double-stranded molecule digestion tends to be "self-limiting", and will decrease when there are no remaining regions of double-stranded DNA. Thus the exonuclease treatment can effectively convert each starting double-stranded DNA molecule into two non-complementary single-stranded DNA molecules corresponding to the 3' or 5' "halves" of the original molecule.

Alternatively with knowledge of the average size of molecules within the double-stranded DNA population (determined, for example, by gel electrophoresis) and the rate of digestion by the chosen exonuclease it is possible to chose an incubation period such that the digestion removes a chosen length of the DNA molecules. This chosen length may, for example, be approximately half the average DNA molecule length present in the starting double-stranded DNA population. Such a digest will, as with the technique described above, produce two single-stranded DNA molecules corresponding to the (3' or 5') "halves" of the lengths of the two starting strands of the original double-stranded DNA.

It will be appreciated that as a result of the digestion the two remaining molecules are not complementary to one another. This therefore prevents the strands of, for example, the probe library molecules (i.e. molecules derived by exonuclease digestion of cDNA from the sample of interest) re-hybridising to their complementary sequences found within the original double stranded DNA population from the sample of interest. Thus these DNA molecules are maintained in single stranded form within the probe library and are therefore free to hybridise to complementary sequences in the reference samples (should such sequences be present), thereby improving the sensitivity of the method of the invention. It is preferred that both the library of probe molecules and the reference samples are prepared using exonuclease digestion as described above.

In practice, in order to maximise sensitivity the specificity of the exonucleases will be reversed for probe and reference samples in order to ensure that probe sequences will complement and therefor efficiently hybridise to reference samples. For example, in one embodiment the probe will be treated with a 3'-5' exonuclease and reference samples treated with a 5'-3' exonuclease. In an alternative embodiment the probe will be treated with a 5'-3' exonuclease and reference samples treated with a 3'-5' exonuclease.

Complexity reduction techniques may also be used in preparation of the probe library and/or reference samples to improve the sensitivity of the method of the invention. The rationale behind such techniques is that many of the mRNAs present in a biological sample, such as the sample of interest or the reference samples, represent transcription of so-called "house keeping" genes. These genes encode products associated with the upkeep of the cell and are generally likely to be common to both samples of interest and reference samples. As such they represent components of gene expression patterns that may be found in both test and reference samples, but which are unlikely to be important in the development or maintenance of a biological condition or state of interest. It has been estimated that up to 65% of mRNA mass within cells may be composed of transcripts representing "house-keeping" genes.

Complexity reduction techniques improve sensitivity either by simply reducing the number of individual genes represented in the probe library and/or reference samples, or by specifically removing irrelevant or "house keeping" genes from the probe library and/or reference samples. Thus the relative abundance of those molecules representative of gene expression that remain after application of a complexity reduction technique is increased, thereby increasing the "signal to noise" ratio.

A number of complexity reduction techniques may be used in effecting the method of the invention. These techniques may be used in isolation or in combination. Preferably the same complexity reduction technique, or combination of complexity reduction techniques, are used to treat the cDNA, or its derivatives, to produce both the probe library and the reference samples, although it is possible to apply complexity reduction techniques to only one of the DNA populations.

Suitable examples of complexity reduction techniques include:

### Restriction enzyme based.

In this complexity reduction technique site specific endonucleases are used to digest the cDNA or its derivatives. Since the frequency of cleavage sites for any specific endonuclease will depend on the size and base composition of the cleavage site endonucleases can be chosen that will cut a sub-set of all DNA molecules present. For example, a restriction endonuclease recognising a six base site will, on average, cleave every 4,096 base pairs. Thus in a DNA population in which the average polynucleotide size is 2,000 bases such a endonuclease will cleave approximately half of all polynucleotides present. Following restriction digestion either the cleaved products or the uncleaved products can be selectively enriched. By choosing the appropriate restriction enzymes distinct subsets of cDNA sequences can be either eliminated or enriched. By applying this type of strategy the initial total cDNA sample can divided into subsets of genes whereby each sequence is effectively enriched making it more likely that changes in each individual gene will be detected during array hybridisation.

Thus for individual gene sequence present after applying complexity reduction there will be an increase in specific activity for each gene and an increase in the "signal to noise".

### Display products.

Another means of selecting a subset of sequences present in the starting cDNA/mRNA population, and thereby increasing the relative abundance of each selected sequence after complexity reduction, is the use of approaches for differential cDNA display (Liang and Pardee, 1992). cDNA display selectively amplifies only those cDNA populations which contain effective priming sites for display primer(s) used. Display primers can be used to prepare distinct subsets of cDNAs directly from starting RNA (Liang and Pardee, 1992) or alternatively display amplification may be applied to amplified total cDNA populations (Candeliere et al., 1999). In essence display techniques reduce complexity by selectively enriching a subset of the sequences present in the original mRNA or cDNA population, thereby increasing the relative abundance of the selected sequences within the resultant population.

### Hybridisation depletion and enrichment.

A variety of DNA/RNA subtraction techniques have been developed to deplete DNA/RNA sequences common to two or more pools of DNA/RNA molecules. DNA/RNA subtraction applied to DNA or RNA copies (either direct copies or amplified products) of the original extracted RNA can be used to reduce complexity by removing sequences.

Suitable DNA/RNA subtraction techniques for use according to the invention are well known. One such method involves the production of a single-stranded cDNA library (the "tracer."), such as the cDNA from which the probe library or reference samples are to be generated, from which it is desired to remove certain sequences. A collection of amplified cDNAs representing the sequences that one wishes to eliminate (the "driver"), such as housekeeping genes, is then allowed to hybridise with the tracer. Double stranded DNA molecules, representing hybrids of the tracer and the driver, may then be removed from the total population of DNA based upon their adhesion to hydroxyapatite. The remaining DNA population comprises single stranded DNA molecules representing the tracer population minus the driver population. This subtracted DNA population may then be further amplified as required.

In further refinements of this method "driver" nucleic acids may be covalently linked to compounds which facilitate the physical separation of "driver" nucleic acids (plus any annealed "tracer") from unhybridised "tracer". The separated populations (i.e. those sequences present only in the "tracer", or those sequences shared by both "tracer" and "driver") may then be enriched or depleted relative to one another. For example, driver nucleic acids may be linked to biotin, such that following hybridization all biotinylated hybrids can by segregated by interaction with immobilised avidin, allowing either subtractive enrichment or positive selection. Suitable protocols are described in Welcher et al., 1986; and Weaver et al., 1999. In alternative, but similar, approaches "driver" nucleic acids may be bound to latex beads (as described in Kuribayashi-Ohta et al., 1993, or magnetic particles (as described in Lopez-Fernandez and del Mazo, 1993; and Schraml et al. 1993).

In one embodiment hybridisation depletion/enrichment protocols can be used to remove "unwanted sequences" present in samples from which the probe library and/or reference samples are derived. The nature of the "unwanted sequences" will depend on the biological samples in question. However, as a general rule, sequences which are expressed at similar levels in diverse samples are, by their very nature, uninformative and tend simply to add to the "background" produced during hybridisation.

It is likely that genes expressed at a similar level in biologically divergent tissues will not be characteristic of a particular tissue, and will instead represent.house-keeping genes. By way of example, it is unlikely that genes expressed at a similar level in tissues as biologically different as heart, lung, spleen and testes will be characteristic of any one of these tissues. Sequential hybridisation enrichment can be used to obtain a "pool" of sequences common to different tissues. The resultant pool will represent genes that contribute to the "background noise" associated with hybridisation. This pool can then be expanded and used to reduce the level of background hybridisation. For example, it is possible to subtract these common sequences from both the probe library and reference samples, thereby reducing the level of total hybridisation. Alternatively it is possible to use the pool of common genes to produce unlabelled competitor DNA and thereby reduce the level of detectable hybridisation.

Using probe libraries and reference samples produced in accordance with the techniques described above the method of the invention may be effected by reference samples and probe library under hybridising conditions. The conditions under which nucleic acids will hybridise to one another are well known to those skilled in the art. Specific conditions are described in greater detail in the accompanying Example. Further examples of conditions suitable for nucleic acid hybridisation can be found in reference works such as "Molecular Cloning: A Laboratory Manual" edited by Maniatis et al.. Other suitable conditions are described in Chee et al. 1996, Iyer et al. 1999, Lipshutz et al. 1995, Lockhart et al. 1995, Schena 1996, Schena et al. 1995, Soares et al. 1997 and Southern 1996.

Similarly, methods for determining the relative degree of hybridisation between populations of nucleic acids are also well known. Methods suitable for effecting the invention include labelling of the probe library with reporters such as fluorescent labels, radioactive labels or chromogenic enzymes. If the reference sample libraries are unlabelled then detection of the chosen label (after removing unbound probe) will confirm the presence of hybridisation between the sample of interest and the reference sample. Suitable techniques for labelling of the molecules comprising the probe library, for detection of hybridised probe and reference DNA molecules and for interpretation of hybridisation data are well known to those skilled in the art. These techniques include those described in Maniatis et al. 1982, Chee et al. 1996, Iyer et al. 1999, Lipshutz et al. 1995, Lockhart et al. 1995, Schena 1996, Schena et al. 1995, Soares et al. 1997 and Southern 1996.

### Use of unlabelled competitor DNA.

When the probe library DNA is labelled and the reference sample DNA is unlabelled the sensitivity of the method of the invention may be improved by the use of unlabelled "competitor" DNA which can compete with the DNA of the probe library for hybridisation with the reference samples. The competitor DNA may be DNA representing common housekeeping genes, or it may be selected DNA representing genes common to the biological sample of interest and/or the reference samples. Since the competitor DNA is unlabelled, hybrids of competitor and reference DNA will not be detected in assessing total hybridisation.

The competitor DNA may be exposed to the reference sample DNA before the addition of the probe library DNA or at the same time as the addition of the probe library DNA. Molecules of the competitor DNA that represent genes expressed by the reference samples will then hybridise to the corresponding DNA of the reference samples. Reference sample molecules that undergo hybridisation with molecules of the competitor DNA will therefore be unable to hybridise with further molecules from the probe library. Thus by incubating the DNA of the reference samples with, for example, unlabelled competitor DNA representative of housekeeping genes it is possible to reduce the level of binding by labelled probe DNA representing the same genes. This therefore improves the sensitivity of the method of the invention since it increases the likelihood that detected hybridisation is representative of genes of interest within the samples.

Unlabelled competitor DNA representative of genes having a high frequency of expression within the biological sample of interest and/or reference samples may be generated by reverse subtraction of the DNA populations derived from the two samples.

The present invention may be used in conjunction with the technique disclosed in our co-pending U.K. patent application (entitled "Analysis of Biological Samples") filed concurrently herewith. This application relates to a method of analysing a biological sample of interest, comprising:
(i) providing a probe library which comprises cDNA or a derivative thereof representative of a pattern of multiple gene expression in the biological sample of interest;
(ii) providing a plurality of individual reference samples each being a library comprised of cDNA or a derivative thereof representative of a pattern of gene expression in reference biological samples from which the reference samples have been derived;
(iii) treating individual reference samples with the probe library under hybridising conditions; and
(iv) determining the relative degree of hybridisation of the probe library to the reference samples, thereby providing an indication of the degree of similarity between gene expression in the biological sample of interest and gene expression in the individual reference biological samples.

The technique disclosed in our co-pending application provides a method by which gene expression within a biological sample of interest may be compared with gene expression in a number of individual biological reference samples by an assessment of the total degree of hybridisation shared between the probe library (representing the sample of interest) and the reference samples. In many situations (depending on the nature of the chosen sample of interest and reference samples) there may be very similar levels of total hybridisation shared between the probe library and a number of different reference samples. In such cases the method of the instant application may be used to provide further, more detailed, information about the degree of similarity shared between the sample of interest and such individual reference samples displaying comparable levels of total hybridisation to the probe library. Thus the method of our co-pending U.K. Patent Application may be used as a "screen" to identify and select reference samples sharing a comparable degree of similarity to the sample of interest (as indicated by comparable levels of total hybridisation). The method of the instant application may then be used to provide further information as to which of the selected reference samples most closely resembles the sample of interest.

A technique utilising the procedure of our co-pending UK Patent Application in combination with the present invention will now be described by way of example only with reference to the accompanying drawing in which:
Figure 1a represents a schematic depiction of an array of reference samples suitable for use in our co-pending U.K. Patent Application before effecting hybridisation;
Figure 1b represents the same array after effecting hybridisation of a probe library with the reference samples;
Figure 1c represents a flow chart indicating suitable methods for producing a probe library and reference samples;
Figure 2 represents a flow chart indicating the processes involved in analysing complexity of hybridisation according to the invention; and
Figure 3 represents a schematic depiction of the use of the method of our co-pending U.K. Patent Application to identify samples to be investigated by the method of the instant application.

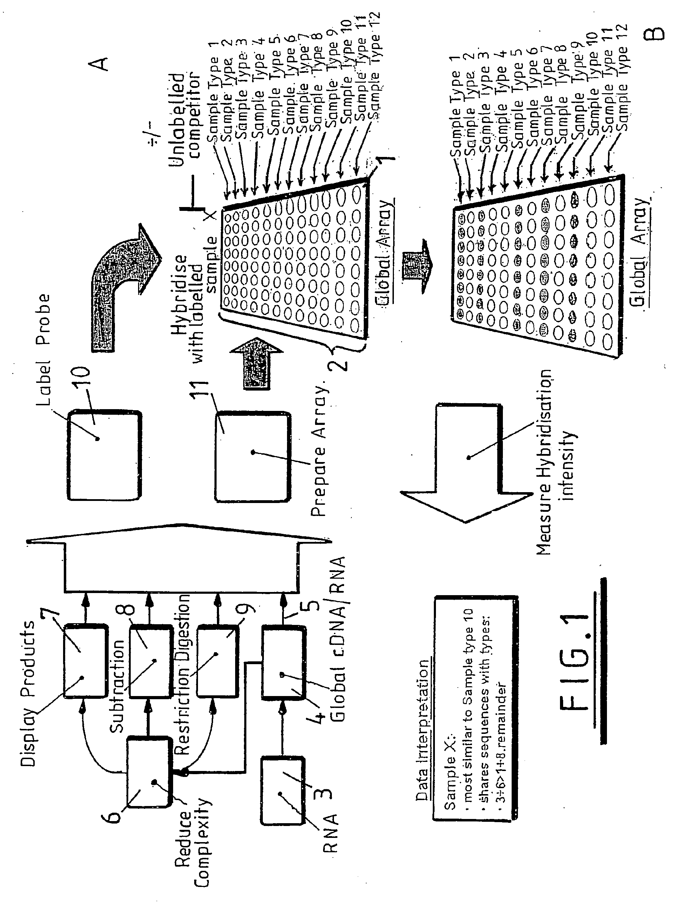

Figure 1a shows an array (1) provided with individual reference samples (2) derived from cDNA generated from biological reference samples. Each individual reference sample is a library representative of a pattern of gene expression in the biological reference sample. The rows of reference samples (2) on the array (1) each represent a distinct biological condition or state. Each reference sample (2) within a row is derived from a different individual sharing the same biological condition or state.

Figure 1b shows the results of probing the reference samples (2) on the array (1) with a labelled probe library according to the method of the invention. Sequences present within both the probe library and the reference samples (2) hybridise to one another. Hybridisation is measured by colour development, hence the greater the degree of hybridisation between the probe library and a reference sample the more intense the colour. Thus in Figure 1b it can be seen that the probe library exhibits the greatest degree of similarity (and so hybridisation) with the reference samples of row 10, a lesser degree of similarity (and hybridisation) with reference samples of rows 3 and 6 and a still lesser degree of similarity with the reference samples of rows 1 and 8. The probe library does not share any sequences in common with the other rows of reference samples (2) and thus does not hybridise with these reference samples, so producing no colour development.

The probe library and reference samples may be prepared by the procedures illustrated in Figure 1c, in which RNA (3) from a biological sample of interest is amplified according to known protocols to generate global cDNA (4). Tins global cDNA (4) may then be used directly to produce a probe library (as indicated by arrow 5) or, more preferably, is subjected to complexity reduction techniques (6) prior to probe library production.

Complexity reduction (6) may, for instance, take the form of processing to display products (7), subtraction of unwanted sequences from the global cDNA generated from the sample of interest (8) or restriction digest of sequences in the cDNA generated from the sample of interest (9). The cDNA generated from the sample of interest may be subject to a combination of complexity reduction techniques (e.g. subtraction (8) and restriction digest (9)) or may be used to produce a probe library after a single complexity reduction technique.

Optionally, the cDNA, or derivative, of the probe library may be subject to exonuclease digestion in order to improve the sensitivity of the invention. This digestion may be effected either before or after complexity reduction.

Production of the probe library is completed by using known techniques to label (10) the cDNA generated from the sample of interest.

Although the generation and processing of cDNA has been described above with reference to production of the probe library, the techniques described (with the exception of labelling the probe library) are all equally suitable for production of reference samples from biological reference samples in order to produce a suitable array (11). Preferably both the probe library and reference samples to be used according to the method of the invention are produced using the same complexity reduction techniques. In the situation that both the probe library and reference samples are to be subject to exonuclease digestion the two different cDNA populations should be treated with exonucleases having different specificities, i.e. one treated with a 5' to 3' exonuclease, and the other treated with a 3' to 5' exonuclease.

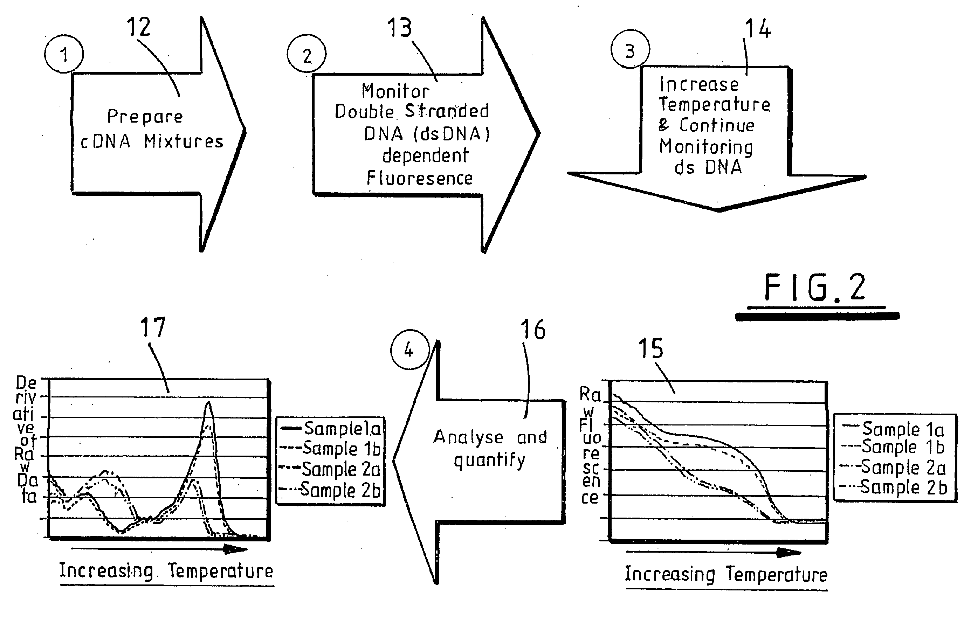

As illustrated in Figure 1, the probe library may sometimes share a similar degree of hybridisation (or the same degree of hybridisation) with two or more references samples. However, whilst the probe library may share the same level of total hybridisation with the reference samples (reflecting the same level of total gene expression in common with the different reference samples) it may share different genes in common with the different samples. In order to determine more information about the genes expressed in common between the sample of interest and reference samples it is possible to perform a complexity analysis technique as illustrated in Figure 2.

In this instance complexity analysis is performed using the cDNA of the probe library separately hybridised with the cDNA of four reference samples (12). These four reference samples (1a, 1b, 2a, 2b) represent duplicate copies of two reference samples exhibiting similar levels of total hybridisation to the probe library.

The hybridised cDNA is labelled with a fluorophore whereby the fluorescence is dependent on the presence of double stranded DNA. The labelled hybridised cDNA is then subjected to incrementally increasing temperature (14) and the fluorescence monitored (13) according to established techniques to assess dissociation of the cDNA brought about by the increase in temperature. Different cDNAs (representing the expression of different genes) will dissociate at different temperatures. These temperatures may be determined experimentally. By analysing the dissociation of the hybridised cDNAs it is thus possible to determine further information as to which genes are represented by the hybridised cDNAs.

Recording the decreasing fluorescence that results from dissociation of the hybridised cDNA produces a graph (15). This depicts the fluorescence on the Y axis and temperature on the X axis. It can be seen that fluorescence decreases as temperature increases, due to dissociation of the cDNA of the probe library from the reference samples. It can further be seen that this decrease in fluorescence occurs at a different rate for each of the four reference samples. Samples 1a and 1b represent independent samples that are related to one another. Samples 2a and 2b are also related to one another, but are not related to samples 1a and 1b. Related samples 1a and 1b produce dissociation curves having similar patterns. The patterns of these curves are different from those of the dissociation curves produced by samples 2a and 2b. However, the dissociation curves produced by related samples 2a and 2b share similar patterns to one another. This indicates that cDNA of the probe library has hybridised with different cDNAs (representing expression of different genes) in the different reference samples.

The data contained in graph 15 can be analysed (16) using commercially available software to produce graph 17. Graph 17 plots the negative of the derivative (gradient) in graph 15 (Y axis) against increasing temperature (X axis). Thus the temperatures causing the greatest decrease in the gradient of the dissociation curves shown in graph 15 (i.e. the largest degree of dissociation) correspond to the greatest peaks on graph 17. The four dissociation curves on graph 17 therefore provide information as to the temperature at which the cDNAs of the four reference samples dissociate from the cDNAs of the probe library. These graphs can be compared to dissociation curves generated by the dissociation of probe library samples that have hybridised to probe library samples, and to dissociation curves produced from hybridised cDNA samples representing known genes, in order to provide an indication as to the degree of similarity and the genes shared between the sample of interest and the reference samples.

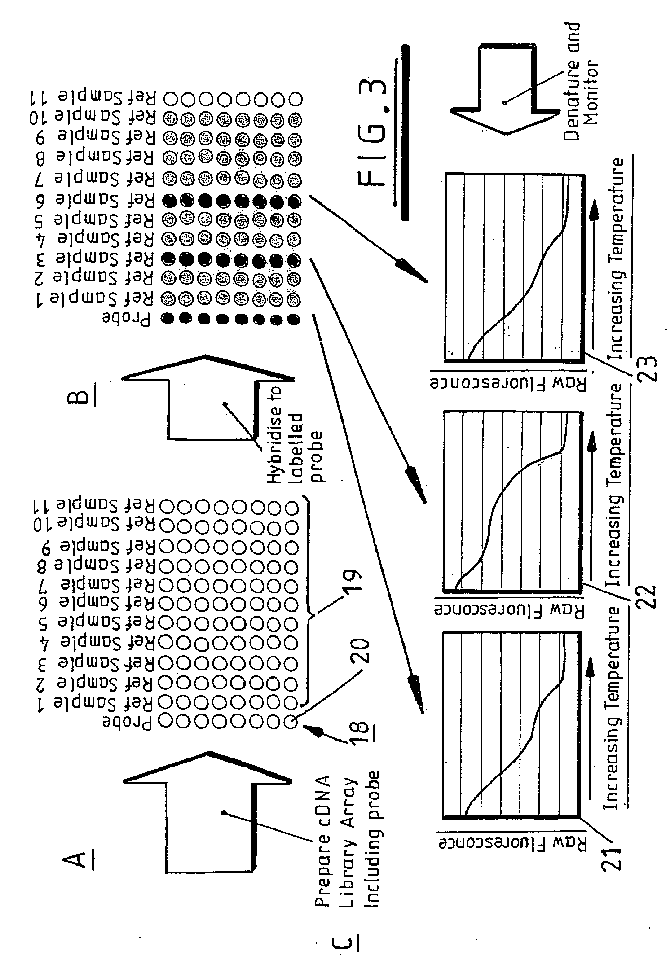

Turning now to Figure 3 a, an array 18, comprises both immobilised reference samples 19 and immobilised probe library samples 20. In Figure 3b the results of hybridisation of the probe library with array 18 are shown, where increasing hybridisation is indicated by increasing intensity of colour. Thus it can be seen that the probe library undergoes the greatest degree of total hybridisation with the immobilised probe library samples 20, and a lesser (but comparable) degree of total hybridisation with reference samples 3 and 6 (indicated as "Ref Sample 3" and "Ref Sample 6").

Figure 3c illustrates dissociation curves 21, 22 and 23 (recording respectively dissociation of the free probe library and: (21) Immobilised probe library; (22) Reference Sample 3; and (23) Reference Sample 6) produced in accordance with the method of the invention. Comparison of the three dissociation curves illustrates that the curve generated on dissociation of the free probe library from the immobilised probe library (curve 21) most closely resembles the curve generated on dissociation of the free probe library from reference sample 6 (curve 23), indicating that the biological sample of interest is most similar to reference sample 6.

### Protocols.

The following Protocols are suitable for effecting the method of the invention.
(a) Preparation of global amplified cDNA
   (i) Preparation of cDNA
   (ii) Terminal transferase - "Tailing"
   (iii) Global cDNA amplification
(b) Preparation of array of reference samples
(c) Labelling of probe library
   (i) Terminal Transferase labelling
   (ii) PCR labelling
(d) Hybridisation of probe library and reference samples
(e) Complexity reduction.
   (i) Display Based
   (ii) Hybridisation depletion and enrichment
(f) Detection of hybridisation
   (i) Removal of hybridised probe
   (ii) Monitoring dsDNA

### (a) Preparation of global amplified cDNA.

The protocol described below is based on protocols described in Brady et al. (1990) and Brady, G., and Iscove, N. N. (1993).

Suitable starting materials include total RNAs, which may be prepared from biological tissues of interest (using commercially available kits such as those manufactured by Clontech), or mRNA present in biological cells ("direct amplification").

### (i) Preparation of cDNA.

cDNA may be prepared from the mRNA from the biological tissues according to the following protocol:
1. RNAs are adjusted to 100 microgram/ml in 10 mM Tris pH 7.5, 1 mM EDTA
2. 3 µl of each RNA is added to 3 µl of the following buffer:

| | |
|---|---|
| 100 mM | Tris pH 8.3 |
| 150 mM | KCl |
| 6 mM | MgCl₂ |
| 0.2 mg/ml | Glycogen (Roche) |
| 2 % | NP-40 (Roche) |
| 2.5 nM | dNTPs (Sigma) |
| 0.75 µM | dT24 (Sigma/Genosys) |
| 0.37 u/ml | RNAse inhibitors (Ambion) |

3. Samples are heated to 65°C for 1 minute allowed to cool at RT for 3 minutes then placed on wet ice
4. After 1 to 10 minutes on ice 3 µl of the following buffer containing 85 u MMLV RTase and 1 u AMV RTase is added to each sample:

| | |
|---|---|
| 50 mM | Tris pH 8.3 |
| 75 mM | KCl |
| 3 mM | MgCl₂ |
| 0.1 mg/ml | Glycogen (Roche) |
| 1 % | NP-40 (Roche) |

5. Samples are Incubated 15 minutes at 37°C, heat inactivated at 65°C for 10 minutes then cooled to 4°C.

### (ii) Terminal Transferase - 'Tailing'

1. 5 µl of each sample is mixed with 5 µl of the following buffer containing 2.3 units terminal transferase.

| | |
|---|---|
| 200 mM | potassium cacodylate pH 7.2 |
| 4 mM | COCl₂ |
| 0.4 mM | DTT |
| 1 mM | dATP |

2. Samples are then incubated 15 minutes at 37oC, 65oC 10 minutes and cooled to 4oC.

### (iii) Global cDNA amplification.

Global cDNA prepared from biological tissues according to the preceding protocols may be amplified according to the following protocol:
1. 8 µl of the tailed cDNA prepared as described above may be combined with 8 µl of:

| | |
|---|---|
| 121.4 mM | KCl |
| 8.5 mM | MgCl₂ |
| 24.25 mM | Tris-HCl pH 8.3 |
| 48 µg/ml | Glycogen (Roche) |
| 2.4 % | Triton X-100 |
| 2.3 mM | dNTPs |
| 9.6 µM | Oligo Not1dT (sequence CATCTCGAGCGGCCGCTTTTTTTTTTTTTTTTTTTTTTTT) |
| 0.16 u/µl | Taq Polymerase |

2. Samples are then placed into a PCR machine and subjected to:
25 cycles
1 minute 94°C
2 minute 42°C
6 minute 72°C
followed by an additional 25 cycles:
1 minute 94°C
1 minute 42°C
2 minute 72°C

3. Following completion of PCR samples are purified using the Millipore 96 well purification system (Millipore MANU 03050) following instructions provided by the manufacturer.

### (b) Preparation of array of reference samples.

An array comprising global purified cDNA (prepared as described above) may be produced using the following protocol:

Purified global cDNAs from heart, lung, spleen and testes may separately be adjusted to around 50 ng/µl in 50% DMSO, boiled and spotted in groups of 12 onto CMTGAPS glass slides (Corning) using a Gene Machines OmniGrid as recommended by the manufacturer.

### (c) Labelling of probe library.

The following provides suitable protocols for labelling of probe library cDNA for use according to the method of the invention. The following protocols describes the labelling of two different cDNA populations (which may be prepared using the protocols described above) with two different fluorescent markers (Cy3 and Cy5).

### (i) Terminal Transferase labelling.

1. Approximately 50 ng of globally amplified cDNA of a first probe library may be added to a 20 µl reaction containing:

| | |
|---|---|
| 100 nM | FluoroLink^{™} Cy3-dUTP (Amersham Pharmacia Biotech) |
| 100 mM | potassium cacodylate pH 7.2 |
| 2 mM | CoCl₂ |
| 0.2 mM | DTT |
| total 5 units | Terminal Transferase |

2. Approximately 50 ng of globally amplified cDNA of a second probe library may be added to a 20 µl reaction containing:

| | |
|---|---|
| 100 nM | FluoroLink^{™} Cy5-dUTP (Amersham Pharmacia Biotech) |
| 100 mM | potassium cacodylate pH 7.2 |
| 2 mM | CoCl₂ |
| 0.2 mM | DTT |
| total 5 units | Terminal Transferase |

3. Following incubation for 1 hour at 37°C both samples may be ethanol precipitated by the addition of:

| | |
|---|---|
| 10 µl | 7.5 M Ammonium Acetate |
| 0.5 µl | 15 mg/ml Glyco Blue (Ambion) |
| 75 µl | ethanol |

Samples may then be held on wet ice for 15minutes, centrifuged at 4°C at 14,000 rpm for 20 minutes and the pellets washed twice with 70% ethanol, allowed to dry 10 minutes at room temperature then resuspended in 5 µl 10 mM Hepes pH 7.5, 1 mM EDTA.

### (ii) PCR labelling.

Further rounds of PCR amplification can be used to incorporate fluorescent markers directly or indirectly coupled to nucleotides present in the PCR reaction. An example of such an approach is given below.
1. Approximately 0.5 ng of globally amplified cDNA of a first probe library may be added to a 20-100 µl reaction containing:

| | |
|---|---|
| 100 nM | FluoroLink^{™} Cy3-dUTP (Amersham Pharmacia Biotech) |
| 100nM | dNTPs |
| 1 µM | Oligo Not1dT (sequence CATCTCGAGCGGCCGCTTTTTTTTTTTTTTTTTTTTTTTT) |
| 16mM | (NH₄)₂SO₄ |
| 67mM | Tris-HCl (pH 8.8 at 25°C) |
| 0.01 % | Tween-20 |
| 0.16 u/µl | Taq Polymerase |

2. Approximately 0.5 ng of globally amplified cDNA of a second probe library may be added to a 20-100 µl reaction containing:

| | |
|---|---|
| 100 nM | FluoroLink^{™} Cy5-dUTP (Amersham Pharmacia Biotech) |
| 100nM | dNTPs |
| 1 µM | Oligo Not1dT (sequence CATCTCGAGCGGCCGCTTTTTTTTTTTTTTTTTTTTTTTT) |
| 16mM | (NH₄)₂SO₄ |
| 67mM | Tris-HCl (pH 8.8 at 25°C) |
| 1.5 mM | MgCl₂ |
| 0.01 % | Tween-20 |
| 0.16 u/µl | Taq Polymerase |

3. Both samples are then placed into a PCR machine and subjected to:

| 25 cycles | |
|---|---|
| 30 seconds | 94°C |
| 1 minute | 42°C |
| 2 minutes | 72°C |

4. Following PCR both samples may be ethanol precipitated by the addition of:

| | |
|---|---|
| 0.5 original sample volume | 7.5 M Ammonium Acetate |
| 0.025 original sample volume | 15 mg/ml Glyco Blue (Ambion) |
| 3.5 original sample volumes | ethanol |

Samples may then be held on wet ice for 15minutes, centrifuged at 4°C at 14,000 rpm for 20 minutes and the pellets washed twice with 70% ethanol, allowed to dry 10 minutes at room temperature then resuspended in 5 µl 10 mM Hepes pH 7.5, 1 mM EDTA.

### (d) Hybridisation of probe library and reference samples.

Hybridisation of probe library and reference samples according to the method of the invention may be effected as follows, using an array and probe libraries prepared as described above.
1. An array slide may be prehybridised at 42°C for 1 hour in the following buffer:
50% Formamide
5X SSC
0.1 % SDS
10 mg/ml BSA

2. The array slide may then be washed four times with H₂O and once in Isopropanol and dried 5 minutes at room temperature.
3. The following mixture may then be prepared:

| | |
|---|---|
| 50%v/v | Formamide |
| 5X | SSC |
| 0.1 % | SDS |
| 0.5mg/ml | Poly A RNA |
| 0.5mg/ml | Yeast tRNA |
| 0.5mg/ml | Salmon Sperm DNA (10-30ug) |
| 50ug/ml | Cotl DNA |

combined Cy3 and Cy 5 probes
(Total volume 45 µl)
4. The mixture may then be heated at 95°C for 5 minutes and chilled on wet ice 3 minutes.
5. The mixture may be applied to a cover slip and the pre-warmed (42°C) array slide (arrayed material facing downwards) lowered onto cover slip to the point when it is just possible to lift the cover slip up with surface tension.
6. The slide may be placed into a moisturised slide hybridisation chamber and incubated 42 °C o/n.(<16hr).
7. Following hybridisation the entire slide may be immersed in 2X SSC and the cover slip removed.
8. The exposed slide may then be washed twice 2X SSC/0.1% SDS (5 minutes RT each wash) followed by 2 washes with 2X SSC (5 minutes RT each wash) and drying at room temperature.

### (e) Complexity reduction.

There are many possible complexity reduction techniques that are suitable for use with the method of the invention.

### (i) Display based

The following protocol is suitable for effecting a "display products" complexity reduction technique according to the method of the invention. The protocol provides for the preparation of two different amplified cDNA populations from the same cDNA population ("total cDNA").

Selected subsets of cDNA within a global amplified total cDNA population may be further amplified based on protocols described in:

Candeliere, G. A., Rao, Y., Floh, A., Sandler, S. D., and Aubin, J. E. (1999). cDNA fingerprinting of osteoprogenitor cells to isolate differentiation stage-specific genes. Nucleic Acids Research 27, 1079-83.

A suitable protocol is as follows:
1. Purified globally amplified total cDNA prepared as described above may be diluted 100 fold in 2 mM Tris pH 7.5, 0.2 mM EDTA.
2. Two separate subsets of cDNAs may then be selectively amplified from the total cDNA by separately adding 10 µl of total cDNA to 10 µl of PCR mixture A and 10 µl of total cDNA to 10 µl of PCR mixture B, and subjecting both to:
2 cycles as follows:

| | |
|---|---|
| 94°C | 1 minutes; |
| 35°C | 3 minutes; |
| 72°C | 3 minutes; |

followed by 30 cycles as follows:

| | |
|---|---|
| 94°C | 30 seconds; |
| 50°C | 30 seconds; |
| 72°C | 1 minute; and |

1 cycle as follows:

| | |
|---|---|
| 72°C | 5 minutes. |

### PCR mixture A

| | |
|---|---|
| 25 µM | Display Oligo A - CAGCCAGTCTTGAGGCAACACC |
| 0.5 mM | dNTPs (Sigma) |
| 32 mM | (NH₄)₂SO₄ |
| 134 mM | Tris-HCl (pH 8.8 at 25°C) |
| 0.01% | Tween-20 |
| 3 mM | MgCl₂ |
| 25 u/ml | Taq Polymerase |

### PCR mixture B

| | |
|---|---|
| 25 µM | Display Oligo B - CCAGCAAGAGCACAAGAGGAAGAG |
| 0.5 mM | dNTPs (Sigma) |
| 32 mM | (NH₄)₂SO₄ |
| 134 mM | Tris-HCl (pH 8.8 at 25°C) |
| 0.01% | Tween-20 |
| 3mM | MgCl₂ |
| 25 u/ml | Taq Polymerase |

Following PCR all samples may be purified using GFX purification columns (Amersham Pharmacia) following the manufacturer's instructions.

### (ii) Hybridisation depletion and enrichment

The term *driver* refers to the cDNA used to deplete and/or enrich in the *tracer* cDNA population. The resultant depleted or enriched sequences will be derived from the *tracer* cDNA population. In the following examples all *driver* cDNAs are prepared in PCR reactions containing dUTP (not dTTP) to allow removal of residual *driver* cDNAs using the dUTP specific UNG nuclease.

### Based on methods described in:

Analysis of gene-expression in a complex differentiation hierarchy by global amplification of cdna from single cells. Brady, G, Billia F, Knox J, Hoang T, Kirsch IR, Voura EB, Hawley RG, Cumming R, Buchwald M, Siminovitch K, Miyamoto N, Boehmelt G, and Iscove NN: *Current Biology* 1995, 5: 909-922.

Foot, HCC, Brady G, and Franklin FCH. (1996). Subtractive Hybridisation. In Plant Molecular Biology Laboratory Manual, M. Clark, ed. (London: Springer Verlag).

Weaver, DL, Núñez C, Brunet C, Bostock V, and Brady G. (1999). Single-cell RT-PCR cDNA subtraction. In Molecular Embryology: Methods and Protocols., P. Sharpe and I. Mason, eds. (Totowa, NJ, USA: Humma Press), pp. 601-609.

### Depletion/Subtraction

### 1. Preparation of tracer and driver:

### Tracer

Approximately 0.5 ng of globally amplified cDNA added to a 20-100 µl reaction containing:

| | |
|---|---|
| 250 nM | dATP, dTTP, dCTP, dGTP |
| 1 µM | Oligo Not1dT (sequence CATCTCGAGCGGCCGCTTTTTTTTTTTTTTTTTTTTTTTT) |
| 16mM | (NH₄)₂SO₄ |
| 67mM | Tris-HCl (pH 8.8 at 25°C) |
| 1.5 mM | MgCl₂ |
| 0.01% | Tween-20 |
| 0.16 u/µl | Taq Polymerase |

### Driver

Approximately 0.5 ng of globally amplified cDNA added to a 20-100 µl reaction containing:

| | |
|---|---|
| 250 nM | dATP, dUTP, dCTP, dGTP |
| 1 µM | Oligo Not1dT (sequence CATCTCGAGCGGCCGCTTTTTTTTTTTTTTTTTTTTTTTT) |
| 16mM | (NH₄)₂SO₄ |
| 67mM | Tris-HCl (pH 8.8 at 25°C) |
| 1.5 mM | MgCl₂ |
| 0.01% | Tween-20 |
| 0.16 u/µl | Taq Polymerase |

Both *tracer* and *driver* are then placed into a PCR machine and subjected to:

| 25 cycles | |
|---|---|
| 30 seconds | 94°C |
| 1 minute | 42°C |
| 2 minutes | 72°C |

Following completion of the PCR reaction both *tracer* and *driver* cDNAs are then purified using commercial purification systems such as GFX (Amersham Pharmacia).

### Biotinylation of Driver.

Place 20-50 µl driver DNA (2-50 µg) in a 1.5 ml screw-cap tube. Boil for 2 minutes and place directly on ice in a small ice tray + rack.

Add 20 µl 2 mg/ml photobiotin to the DNA and mix well. With the lids left off place the tubes upright on ice 10 cm from the bulb and irradiate for a total 10 minutes. After the first 5 minutes remove the tubes from under the light source (avoid direct irradiation), flick the tube to mix and replace under the light source for the remaining 5 minutes.

Remove the sample (avoid direct irradiation) and mix in the remaining 20 µl of: photobiotin and place under the light for an additional 5 minutes.

Add 1/10th volume of 1M Tris-Cl, pH 8.0 to stop the reaction.

Purify using commercial purification systems such as GFX (Amersham Pharmacia).

### 2. Hybridisation of tracer plus driver and tracer enrichment:

To a 0.5 ml tube add and mix in this order:

| | |
|---|---|
| 0.5 µg | *tracer DNA* |
| 10 µg | biotinylated *driver DNA* |

adjust volume to 20 µl with water then add:

| | |
|---|---|
| 8 µl | 5xHyb *GEH* |
| 12 µl | 40 % PEG |

Heat sample:
5 minutes 98°C,
5 minutes at 80°C
7 minutes at 74°C
60 minutes at 68°C
then hold at 68°C while seperating biotinylated molecules

Remove biotinylated molecules using avidin bound to a solid support. In practise this can be carried out using commercial products as ditrected by the manufacturer such as Streptavidin Magnasphere^{™} Paramagnetic particles (SA-PMPs) provided by Promega.

Following removal of biotinylated molecules the remaining *tracer* can be subjected to further rounds of subtraction by addition of fresh biotinylated *driver DNA* and repeating the process described above. Typically three sequential rounds of subtraction are used but additional rounds may be added if required.

The final depleted product is then amplified using PCR conditions described for the original tracer amplification.

### 5xHyb GEH

| | |
|---|---|
| 90 mM | EPPS pH 8.5 |
| 10 mM | EDTA pH 8.0 |
| 0.5 % | Triton X-100 |
| 3.75 M | NaCl |

### Negative Subtraction or Attraction

### 1. Preparation of tracer and driver:

### Tracer

Approximately 0.5 ng of globally amplified cDNA added to a 20-100 µl reaction containing:

| | |
|---|---|
| 250 nM | dATP, dTTP, dCTP, dGTP |
| 1 µM | Oligo Not1dT (sequence CATCTCGAGCGGCCGCTTTTTTTTTTTTTTTTTTTTTTTT) |
| 16mM | (NH₄)₂SO₄ |
| 67mM | Tris-HCl (pH 8.8 at 25°C) |
| 1.5 mM | MgCl₂ |
| 0.01% | Tween-20 |
| 0.16 u/µl | Taq Polymerase |

### Driver

Approximately 0.5 ng of globally amplified cDNA added to a 20-100 µl reaction containing:

| | |
|---|---|
| 250 nM | dATP, dUTP, dCTP, dGTP |
| 1 µM | Oligo Not1dT (sequence CATCTCGAGCGGCCGCTTTTTTTTTTTTTTTTTTTTTTTT) |
| 16mM | (NH₄)₂SO₄ |
| 67mM | Tris-HCl (pH 8.8 at 25°C) |
| 1.5mM | MgCl₂ |
| 0.01 % | Tween-20 |
| 0.16 u/µl | Taq Polymerase |

Both *tracer* and *driver* are then placed into a PCR machine and subjected to:

| 25 cycles | |
|---|---|
| 30 seconds | 94°C |
| 1 minute | 42°C |
| 2 minutes | 72°C |

Following completion of the PCR reaction both *tracer* and *driver* cDNAs are then purified using commercial purification systems such as GFX (Amersham Pharmacia).

### Biotinylation of Driver

Place 20-50 µl driver DNA (2-50 µg) in a 1.5 ml screw-cap tube. Boil for 2 minutes and place directly on ice in a small ice tray + rack.

Add 20 µl 2 mg/ml photobiotin to the DNA and mix well. With the lids left off place the tubes upright on ice 10 cm from the bulb and irradiate for a total 10 minutes. After the first 5 minutes remove the tubes from under the light source (avoid direct irradiation), flick the tube to mix and replace under the light source for the remaining 5 minutes.

Remove the sample (avoid direct irradiation) and mix in the remaining 20 µl of photobiotin and place under the light for an additional 5 minutes.

Add 1/10th volume of 1M Tris-Cl, pH 8.0 to stop the reaction.

Purify using commercial purification systems such as GFX (Amersham Pharmacia).

### 2. Hybridisation of tracer plus driver and tracer enrichment:

To a 0.5 ml tube add and mix in this order:

| | |
|---|---|
| 0.5-10 µg | *tracer DNA* |
| 10 µg | biotinylated *driver DNA 1* |

adjust volume to 20 µl with water then add:

| | |
|---|---|
| 8 µl | 5xHyb *GEH* |
| 12 µl | 40 % PEG |

Heat sample:
5 minutes 98°C,
5 minutes at 80°C
7 minutes at 74°C
60 minutes at 68°C
then hold at 68°C while seperating biotinylated molecules

Remove biotinylated molecules using avidin bound to a solid support. In practise this can be carried out using commercial products as directed by the manufacturer such as Streptavidin Magnasphere^{™} Paramagnetic particles (SA-PMPs) provided by Promega.

Release *tracer DNA* bound to *driver DNA 1* by denaturing the *driver DNA 1*/*tracer DNA* hybrids. For examples using SA-PMPs the washed SA-PMPs and their attendant *driver DNA1*/*tracer DNA* hybrids can be heated to 96°C to release *tracer DNA* and bound *driver DNA 1* removed by magnetic attraction of the SA-PMPs.

Released *tracer DNA* can then be subjected to further rounds of attraction by repeating the process with separate drivers (*driver DNAs 2, 3, 4* etc).

The final "attracted" product will be enriched for sequences common to all *driver DNAs* used and can be amplified using PCR conditions described for the original tracer amplification.

### 5xHyb GEH

| | |
|---|---|
| 90 mM | EPPS pH 8.5 |
| 10 mM | EDTA pH 8.0 |
| 0.5 % | Triton X-100 |
| 3.75 M | NaCl |

### (f) Detection of hybridisation.

### (i) Removal of hybridised probe

The following protocol is suitable for detection and analysis of hybridisation in the method of the invention.
1. Scanning of the slide and quantification of red (Cy5 635nm) and green (Cy3 532mn) fluorescence may be carried out using a GenePix 4000b as recommended by the manufacturer.
2. Following scanning data may be analysed using commercially available software.
3. Additional scans of the same hybridised set may also be made following an increased stringency wash which will partially denature and remove subsets of sequences.
4. Assemble collected data from sequential scans carried out with increasing stringency/denaturation to create a denaturation curve for each immobilised sample.

### (ii) Monitoring dsDNA

The following protocol is suitable for monitoring the extent of ssDNA during
1. Starting material can be either total cDNA or complexity reduced material (see sections a and e).
2. Hybridise and select of hybrids as described in the ***Negative Subtraction*** in section d with the addition of Sybr Green I to the hybridisation reaction (1:66000 of Sybr Green I stock provided with qPCR^{™} Core Kit for Sybr^{™} Green I from Eurogentec).
3. Following selction of hybrids adjust samples to 1X qPCR^{™} Sybr^{™} Green I Buffer (provided with qPCR^{™} Core Kit for Sybr^{™} Green I from Eurogentec).
3. Run denaturation profile on ABI 7000 Sequence Detection System (Applied Biosystems) while monitoring Sybr^{™} Green I fluorescence. A typical denaturation profile would be one that monitors the Sybr^{™} Green I fluorescence while increasing the temperature from 60°C to 95°C.
4. Analyse resultant denaturation curves with either Excel^{™} (Microsoft) or ABI software - 'Dissociation Curve Analysis' program (Applied Biosystems). Typical analysis would involve plotting the negative of the rate of change in fluorescence as a function of temperature.

### References:

Brady, G., Barbara, M., and Iscove, N. N. (1990). Representative in vitro cDNA amplification from individual hemopoietic cells and colonies. Meth. Mol. Cell. Biol. 2, 17-25.
Brady, G., and Iscove, N. N. (1993). Construction of cDNA libraries from single cells. Methods Enzymol. *225,* 611-623.
Chee, M., Yang, R., Hubbell, E., Berno, A., Huang, X. C., Stern, D., Winkler, J., Lockhart, D. J., Morris; M. S., and Fodor, S. P. (1996). Accessing genetic information with high-density DNA arrays. Science *274,* 610-4.
Cumano, A., Paige, D.J., Iscove, N.N. and Brady, G. (1992) Bipotential precursors of B cells and macrophages in murine fetal liver. Nature, 356, 612-615.
Iyer, V. R., Eisen, M. B., Ross, D. T., Schuler, G., Moore, T., Lee, J. C., Trent, J. M., Staudt, L. M., Hudson, J., Jr., Boguski, M. S., Lashkari, D., Shalon, D., Botstein, D., and Brown, P. O. (1999). The transcriptional program in the response of human fibroblasts to serum. Science *283*, 83-7
Kuribayashi-Ohta, K, Tamatsukuri S, Hikata M, Miyamoto C, and Furuichi Y "Application of oligo(dT)30-latex for rapid purification of poly(A)+ mRNA and for hybrid subtraction with the in situ reverse transcribed cDNA.": *Biochim Biophys Acta* 1993, **1156:** 204-12)
Liang, P., and Pardee, A. B. (1992). Differential display of eukaryotic messenger RNA by means of the polymerase chain reaction. Science *257,* 967-71.
Lipshutz, R. J., Morris, D., Chee, M., Hubbell, E., Kozal, M. J., Shah, N., Shen, N., Yang, R., and Fodor, S. P. (1995). Using oligonucleotide probe arrays to access genetic diversity. Biotechniques *19,* 442-7.
Lockhart, D. J., Dong, H., Byrne, M. C., Follettie, M. T., Gallo, M. V., Chee, M. S., Mittmann, M., Wang, C., Kobayashi, M., Horton, H., and Brown, E. L. (1996). Expression monitoring by hybridization to high-density oligonucleotide arrays. Nat Biotechnol *14,* 1675-80.
Lopez-Fernandez, LA, and del Mazo J "Construction of subtractive cDNA libraries from limited amounts of mRNA and multiple cycles of subtraction.": *Biotechniques* 1993, **15**: 654-6, 658-9.
Maniatis, T., Fritsch, E. F., and Sambrook, J. (1982). Molecular Cloning: A Laboratory Manual (Cold Spring Harbor, NY: Cold Spring Harbor University Press).
Schena, M. (1996). Genome analysis with gene expression microarrays. Bioessays *18*, 427-31.
Schena, M., Shalon, D., Davis, R. W., and Brown, P. O. (1995). Quantitative monitoring of gene expression patterns with a complementary DNA microarray. Science *270,* 467-70.
Schraml, P, Shipman R, Stulz P, and Ludwig CU "cDNA subtraction library construction using a magnet-assisted subtraction technique (MAST).": *Trends Genet* 1993, **9:** 70-1.
Sigal, N, Delius H, Kornberg T, Gefter ML, and Alberts B "A DNA-unwinding protein isolated from Escherichia coli: its interaction with DNA and with DNA polymerases.": *Proc Natl Acad Sci USA* 1972, **69:** 3537-41.
Soares, M. B. (1997). Identification and cloning of differentially expressed genes. Curr Opin Biotechnol 8, 542-6.
Southern, E. M. (1996). DNA chips: analysing sequence by hybridization to oligonucleotides on a large scale. Trends Genet *12,* 110-5.
Weaver, DL, Núñez C, Brunet C, Bostock V, and Brady G. (1999). "Single-cell RT-PCR cDNA subtraction. In Molecular Embryology: Methods and Protocols.", P. Sharpe and I. Mason, eds. (Totowa, NJ, USA: Humana Press), pp. 601-609).
Welcher, AA, Torres AR, and Ward DC "Selective enrichment of specific DNA, cDNA and RNA sequences using biotinylated probes, avidin and copper-chelate agarose.": *Nucleic Acids Res* 1986, **14:** 10027-44;
Williams, KR, Spicer EK, LoPresti MB, Guggenheimer RA, and Chase JW "Limited proteolysis studies on the Escherichia coli single-stranded DNA binding protein. Evidence for a functionally homologous domain in both the Escherichia coli and T4 DNA binding proteins.": *J Biol Chem* 1983, **258:** 3346-55.

## Claims

1. A method of determining the degree of similarity between gene expression in a biological sample of interest and that in individual reference samples, comprising
(a) providing a nucleic acid probe library representative of a pattern of gene expression in the biological sample of interest,
(b) providing a plurality of reference samples each being a nucleic acid library representative of a pattern of gene expression in reference biological samples from which the reference samples have been derived,
(c) forming a first set of immobilised, hybridised products by treating the individual reference samples with the probe library under hybridising conditions, one or other of the reference samples or the probe library being in immobilised form, and removing non-immobilised material,
(d) forming a second immobilised product by treating a sample of the free probe library with an immobilised sample of the probe library under hybridising conditions, and removing non-immobilised material,
(e) effecting progressive dissociation of the hybridised products obtained in steps (c) and (d),
(f) monitoring said progressive dissociation, and
(g) comparing the results of step (f) for the hybridised products obtained in step (c) with those obtained for the hybridised products obtained in step (d) to determine said degree of similarity.

2. A method according to claim 1, wherein dissociation of the hybridised samples is brought about by exposing the samples to increasing temperature.

3. A method according to claim 1 or claim 2, wherein dissociation of the hybridised samples is brought about by exposing the samples to increasing concentrations of chemical denaturants.

4. A method according to any preceding claim wherein dissociation is monitored using a marker capable of differentiating between double-stranded and single-stranded nucleic acids.

5. A method according to claim 4, wherein dissociation is monitored using ethidium bromide.

6. A method according to claim 4, wherein dissociation is monitored using SybrGreen.

7. A method according to any preceding claim, wherein dissociation is monitored by detecting the generation of single stranded nucleic acids on dissociation of double stranded hybridised material.

8. A method according to any preceding claim, wherein dissociation is monitored using probe library and reference samples labelled with markers capable of generating a signal when the markers are in proximity to one another that can be distinguished from that signal generated when the markers are distant from one another.

9. A method according to any preceding claim, wherein dissociation is monitored using a labelled non-immobilised nucleic acid population and an unlabelled immobilised nucleic acid population.

10. A method according to claim 9, wherein dissociation is monitored by assessing the residual label retained by the immobilised material on removal of non-immobilised material.

11. A method according to claim 9, wherein dissociation is monitored by assessing the labelled material released from the immobilised material.

12. A method according to any of claims 4 to 11, wherein dissociation is monitored using a fluorescent marker.

13. A method according to any preceding claim, wherein the reference samples are provided as an array on a substrate.

14. A method according to any preceding claim, wherein the reference samples comprise cDNA or a derivative thereof derived from biological reference samples representing a number of different biological conditions or states.

15. A method according to any preceding claim, wherein the reference samples comprise cDNA or a derivative thereof derived from biological reference samples representing a number of different examples of the same biological condition or state.

16. A method according to any preceding claim, wherein the probe library is prepared by a complexity reduction technique from cDNA obtained from the biological sample of interest.

17. A method according to any preceding claim, wherein the reference samples are prepared by a complexity reduction technique from cDNA obtained from the reference biological samples.

18. A method as claimed in claim 16 or claim 17, wherein the complexity reduction technique comprises a restriction digestion technique.

19. A method as claimed in claim 16 or claim 17, wherein the complexity reduction technique comprises a subtraction technique.

20. A method as claimed in claim 16 or claim 17, wherein the complexity reduction technique comprises a cDNA display technique.

21. A method as claimed in any preceding claim, wherein the hybridisation is effected in.the presence of competitor DNA.

22. A method according to any preceding claim, wherein the probe library is labelled with a fluorophore in order to determine the relative degree of hybridisation of the probe library to the reference samples.

23. A method according to any preceding claim, wherein the probe library or reference samples are subject to partial exonuclease digestion prior to effecting hybridisation.

24. A method according to claim 23, wherein both the probe library and the reference samples are subject to partial exonuclease digestion prior to effecting hybridisation, and the probe library and reference samples are treated with exonucleases having different specificities.

## Patentansprüche

1. Verfahren zum Bestimmen des Ähnlichkeitsgrades zwischen Genexpression in einer zu untersuchenden biologischen Probe und derjenigen von individuellen Referenzproben, welches Verfahren umfasst:
(a) Bereitstellen einer Nucleinsäuresondenbank, die für ein Muster der Genexpression in der zu untersuchenden biologischen Probe repräsentativ ist,
(b) Bereitstellen einer Mehrzahl von Referenzproben, bei denen es sich jeweils um eine Nucleinsäurebank handelt, die für ein Muster der Genexpression in biologischen Referenzproben repräsentativ ist, von denen die Referenzproben deriviert worden sind,
(c) Erzeugen einer ersten Reihe von immobilisierten, hybridisierten Produkten durch Behandlung der individuellen Referenzproben mit der Sondenbank unter hybridisierenden Bedingungen, wobei die eine oder andere Referenzproben oder die Sondenbank in immobilisierter Form vorliegt, und Entfernen von nicht-immobilisiertem Material,
(d) Erzeugen eines zweiten immobilisierten Produktes durch Behandeln einer Probe der freien Sondenbank mit einer immobilisierten Probe der Sondenbank unter hybridisierenden Bedingungen und Entfernen von nicht-immobilisiertem Material,
(e) Herbeiführen einer progressiven Dissoziation der in den Schritten (c) und (d) erhaltenen hybridisierten Produkte.
(f) Monitoring der progressiven Dissoziation und
(g) Vergleichen der resultierenden Schritte (f) der hybridisierten Produkte, die in Schritt (c) erhalten wurden, mit denen, die für die hybridisierten Produkte erhalten wurden, die in Schritt (d) erhalten wurden, um den Ähnlichkeitsgrad zu bestimmen.

2. Verfahren nach Anspruch 1, wobei die Dissoziation der hybridisierten Proben durch Exponieren der Proben bei steigender Temperatur herbeigeführt wird.

3. Verfahren nach Anspruch 1 oder 2, wobei die Dissoziation der hybridisierten Proben durch Exponieren der Proben an erhöhten Konzentrationen von chemischen Denaturierungsmitteln herbeigeführt wird.

4. Verfahren nach einem der vorgenannten Ansprüche, wobei die Dissoziation unter Verwendung eines Markers einem Monitoring unterzogen wird, der zur Differenzierung zwischen doppelsträtrgigen und einsträngigen Nucleinsäuren in der Lage ist.

5. Verfahren nach Anspruch 4, wobei die Dissoziation unter Verwendung von Ethidiumbromid einem Monitoring unterzogen wird.

6. Verfahren nach Anspruch 4, wobei die Dissoziation unter Verwendung von SybrGreen einem Monitoring unterzogen wird.

7. Verfahren nach einem der vorgenannten Ansprüche, wobei die Dissoziation einem Monitoring unterzogen wird, indem die Erzeugung einsträngiger Nucleinsäure bei der Dissoziation von doppelsträngigem hybridisierten Material detektiert wird.

8. Verfahren nach einem der vorgenannten Ansprüche, wobei die Dissoziation einem Monitoring unterzogen wird, indem die Sondenbank und mit Markern markierte Referenzproben verwendet werden, die zur Erzeugung eines Signals in der Lage sind, wenn sich die Marker in engem Abstand zueinander befinden, der von dem erzeugten Signal unterschieden werden kann, wenn sich die Marker in einem entfernten Abstand voneinander befinden.

9. Verfahren nach einem der vorgenannten Ansprüche, wobei die Dissoziation einem Monitoring unterzogen wird, indem eine markierte, nicht-immobilisierte Nucleinsäurepopulation und eine nicht-markierte, immobilisierte Nucleinsäurepopulation verwendet werden.

10. Verfahren nach Anspruch 9, wobei die Dissoziation einem Monitoring unterzogen wird, in dem der Restmarker ausgewertet wird, der von dem immobilisierten Material bei Entfernung von nicht-immobilisiertem Material zurückbehalten wird.

11. Verfahren nach Anspruch 9, wobei die Dissoziation einem Monitoring unterzogen wird, indem das von dem immobilisierten Material freigesetzte markierte Material ausgewählt wird.

12. Verfahren nach einem der Ansprüche 4 bis 11, wobei die Dissoziation einem Monitoring unterzogen wird, indem ein Fluoreszensmarker verwendet wird.

13. Verfahren nach einem der vorgenannten Ansprüche, wobei die Referenzproben auf einem Substrat als eine Matrix bereitgestellt werden.

14. Verfahren nach einem der vorgenannten Ansprüche, wobei die Referenzproben cDNA oder ein Derivat davon aufweisen, deriviert von biologischen Referenzproben, die eine Reihe von unterschiedlichen biologischen Bedingungen oder Zuständen repräsentieren.

15. Verfahren nach einem der vorgenannten Ansprüche, wobei die Referenzproben cDNA oder ein Derivat davon aufweisen, deriviert von biologischen Referenzproben, die eine Reihe unterschiedlicher Beispiele der gleichen biologischen Bedingung oder des gleichen biologischen Zustandes repräsentieren.

16. Verfahren nach einem der vorgenannten Ansprüche, wobei die Sondenbank hergestellt wird mit Hilfe einer Methode der Komplexitätsreduktion aus cDNA, die von der zu untersuchenden biologischen Probe erhalten wird.

17. Verfahren nach einem der vorgenannten Ansprüche, wobei die Referenzproben hergestellt werden mit Hilfe einer Methode der Komplexitätsreduktion aus cDNA, die aus den biologischen Referenzproben erhalten wird.

18. Verfahren nach Anspruch 16 oder 17, wobei die Methode der Komplexitätsreduktion eine Methode der Restriktionsdigestion umfasst.

19. Verfahren nach Anspruch 16 oder 17, wobei die Methode der Komplexitätsreduktion eine Subtraktionsmethode umfasst.

20. Verfahren nach Anspruch 16 oder 17, wobei die Methode der Komplexitätsreduktion eine cDNA-Displaymethode umfasst.

21. Verfahren nach einem der vorgenannten Ansprüche, wobei die Hybridisierung in Gegenwart einer kompetitiven DNA herbeigeführt wird.

22. Verfahren nach einem der vorgenannten Ansprüche, wobei die Sondenbank mit einem Fluorphor markiert ist, um den relativen Hybridisierungsgrad der Sondenbank in Bezug auf die Referenzproben zu bestimmen.

23. Verfahren nach einem der vorgenannten Ansprüche, wobei die Sondenbank oder Referenzproben einer partiellen Exonucleasedigestion unterworfen werden, um eine Hybridisierung herbeizuführen.

24. Verfahren nach Anspruch 23, wobei sowohl die Sondenbank als auch die Referenzproben vor der Herbeiführung der Hybridisierung einer partiellen Exonucleasedigestion unterworfen werden und die Sondenbank und Referenzproben mit Exonucleasen behandelt werden, die unterschiedliche Spezifitäten haben.

## Revendications

1. Procédé de détermination du degré de similitude entre l'expression génique dans un échantillon biologique d'intérêt et celle dans des échantillons individuels de référence, comprenant
(a) la fourniture d'une bibliothèque de sondes d'acides nucléiques représentatifs d'un schéma d'expression génique dans l'échantillon biologique d'intérêt,
(b) la fourniture d'une pluralité d'échantillons de référence chacun étant une bibliothèque d'acides nucléiques représentatifs d'un schéma d'expression génique dans les échantillons biologiques de référence à partir desquels les échantillons de référence sont dérivés,
(c) la formation d'un premier ensemble de produits hybridés, immobilisés, par le traitement des échantillons individuels de référence avec la bibliothèque de sondes sous des conditions d'hybridation, l'un ou l'autre des échantillons de référence ou de la bibliothèque de sondes se trouvant sous forme immobilisée, et l'élimination du matériau non immobilisé,
(d) la formation d'un second produit immobilisé par le traitement d'un échantillon de la bibliothèque de sondes libres avec un échantillon immobilisé de la bibliothèque de sondes sous des conditions d'hybridation, et l'élimination du matériau non immobilisé,
(e) l'exécution de la dissociation progressive des produits hybridés obtenus aux étapes (c) et (d),
(f) la surveillance en continu de ladite dissociation progressive, et
(g) la comparaison des résultats de l'étape (f) pour les produits hybridés obtenus à l'étape (c) avec ceux obtenus pour les produits hybridés obtenus à l'étape (d) pour déterminer ledit degré de similitude.

2. Procédé selon la revendication 1, dans lequel la dissociation des échantillons hybridés est provoquée par l'exposition des échantillons à une température croissante.

3. Procédé selon la revendication 1 ou la revendication 2, dans lequel la dissociation des échantillons hybridés est provoquée par l'exposition des échantillons à des concentrations croissantes d'agents dénaturants chimiques.

4. Procédé selon l'une quelconque des revendications précédentes, dans lequel la dissociation est surveillée en continu en utilisant un marqueur capable d'effectuer une différenciation entre des acides nucléiques double brin et simple brin.

5. Procédé selon la revendication 4, dans lequel la dissociation est surveillée en continu en utilisant du bromure d'éthidium.

6. Procédé selon la revendication 4, dans lequel la dissociation est surveillée en continu en utilisant du SybrGreen.

7. Procédé selon l'une quelconque des revendications précédentes, dans lequel la dissociation est surveillée en continu par la détection de la génération d'acides nucléiques simple brin lors de la dissociation du matériau hybridé double brin.

8. Procédé selon l'une quelconque des revendications précédentes, dans lequel la dissociation est surveillée en continu en utilisant la bibliothèque de sondes et les échantillons de référence marqués avec des marqueurs capables de générer un signal lorsque les marqueurs se trouvent à proximité l'un de l'autre qui peut être distingué du signal généré lorsque les marqueurs sont distants l'un de l'autre.

9. Procédé selon l'une quelconque des revendications précédentes, dans lequel la dissociation est surveillée en continu en utilisant une population d'acides nucléiques non immobilisés marqués et une population d'acides nucléiques immobilisés non marqués.

10. Procédé selon la revendication 9, dans lequel la dissociation est surveillée en continu en évaluant le marqueur résiduel retenu par le matériau immobilisé lors de l'élimination du matériau non immobilisé.

11. Procédé selon la revendication 9, dans lequel la dissociation est surveillée en continu en évaluant le matériau marqué libéré du matériau immobilisé.

12. Procédé selon l'une quelconque des revendications 4 à 11, dans lequel la dissociation est surveillée en continu en utilisant un marqueur fluorescent.

13. Procédé selon l'une quelconque des revendications précédentes, dans lequel les échantillons de référence sont fournis sous la forme d'un jeu ordonné d'échantillons sur un substrat.

14. Procédé selon l'une quelconque des revendications précédentes, dans lequel les échantillons de référence comprennent de l'ADNc ou un dérivé de celui-ci dérivé d'échantillons biologiques de référence représentant de nombreu(x)(euses) conditions ou états biologiques différent(e)s.

15. Procédé selon l'une quelconque des revendications précédentes, dans lequel les échantillons de référence comprennent de l'ADNc ou un dérivé de celui-ci dérivé d'échantillons biologiques de référence représentant de nombreux exemples différents de la même condition ou du même état biologique.

16. Procédé selon l'une quelconque des revendications précédentes, dans lequel la bibliothèque de sondes est préparée par une technique de réduction de complexité à partir de l'ADNc obtenu à partir de l'échantillon biologique d'intérêt.

17. Procédé selon l'une quelconque des revendications précédentes, dans lequel les échantillons de référence sont préparés par une technique de réduction de complexité à partir de l'ADNc obtenu à partir des échantillons biologiques de référence.

18. Procédé tel que revendiqué dans la revendication 16 ou la revendication 17, dans lequel la technique de réduction de complexité comprend une technique de digestion par enzyme de restriction.

19. Procédé tel que revendiqué dans la revendication 16 ou la revendication 17, dans lequel la technique de réduction de complexité comprend une technique de soustraction.

20. Procédé tel que revendiqué dans la revendication 16 ou la revendication 17, dans lequel la technique de réduction de complexité comprend une technique de visualisation de l'ADNc.

21. Procédé tel que revendiqué dans l'une quelconque des revendications précédentes, dans lequel l'hybridation est effectuée en présence d'ADN compétiteur.

22. Procédé selon l'une quelconque des revendications précédentes, dans lequel la bibliothèque de sondes est marquée à l'aide d'un fluorophore afin de déterminer le degré relatif d'hybridation de la bibliothèque de sondes aux échantillons de référence.

23. Procédé selon l'une quelconque des revendications précédentes, dans lequel la bibliothèque de sondes ou les échantillons de références sont soumis à la digestion partielle par exonucléase avant d'effectuer l'hybridation.

24. Procédé selon la revendication 23, dans lequel à la fois la bibliothèque de sondes et les échantillons de référence sont soumis à la digestion partielle par exonucléase avant d'effectuer l'hybridation, et la bibliothèque de sondes et les échantillons de référence sont traités avec des exonucléases ayant des spécificités différentes.
